(19) Europäisches Patentamt
European Patent Office
Office européen des brevets

(11) Numéro de publication: **0 007 824**
**B1**

(12) # FASCICULE DE BREVET EUROPEEN

(45) Date de publication du fascicule de brevet: **22.07.81**

(21) Numéro de dépôt: **79400432.5**

(22) Date de dépôt: **27.06.79**

(51) Int. Cl.[3]: **C 07 D 405/04,**
C 07 D 405/14,
C 07 D 413/14,
A 61 K 31/395
//C07D307/79

(54) **Nouveaux dérivés de benzofuranne, leur procédé de préparation et composition thérapeutique les contenant.**

(30) Priorité: **17.07.78 FR 7821188**

(43) Date de publication de la demande:
**06.02.80 Bulletin 80/3**

(45) Mention de la délivrance du brevet:
**22.07.81 Bulletin 81/29**

(84) Etats Contractants Désignés:
**BE CH DE FR GB IT NL SE**

(56) Documents cités:
**GB - A - 1 140 188**

(73) Titulaire: **LABORATOIRES JACQUES LOGEAIS**
**Société dite:**
**71, Avenue du Général de Gaulle**
**F-92130 Issy-Les-Moulineaux (FR)**

(72) Inventeur: **Maillard, Jacques Georges**
**82 bis, Avenue de Paris**
**F-78000 Versailles (FR)**
Inventeur: **Legeai, Jacky**
**3 Résidence Marceau**
**F-91120 Palaiseau (FR)**

(74) Mandataire: **Polus, Camille et al,**
**c/o Cabinet Lavoix 2, Place d'Estienne d'Orves**
**F-75441 Paris Cedex 09 (FR)**

**EP 0 007 824 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouveaux dérivés de benzofuranne, leur procédé de préparation et composition thérapeutique les contenant

La présente invention concerne de nouveaux dérivés du benzofuranne, leur procédé de préparation et leur application en thérapeutique.

La présente invention a pour objet des composés de formule générale:

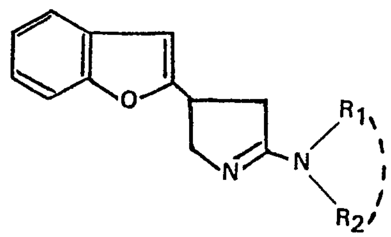

(I)

dans laquelle $R_1$ est une atome d'hydrogène et $R_2$ un atome d'hydrogène, un radical alcoyle en $C_1$—$C_6$, alcényle en $C_2$—$C_6$, alcynyle en $C_2$—$C_6$, phényle, phénylalcoyle ($C_1$—$C_6$), mono, di ou tri-méthoxyphénylalcoyle ($C_1$—$C_6$), alcoxy en $C_1$—$C_6$, benzyloxy, dialcoyl ($C_1$—$C_6$) amino-alcoyle ($C_1$—$C_6$), hydroxyalcoyle ($C_1$—$C_6$), alcoyl ($C_1$—$C_6$) oxycarbonyl alcoyle ($C_1$—$C_6$) ou bien $R_1$ et $R_2$ sont des radicaux alcoyle en $C_1$—$C_6$ ou bien $R_1$ et $R_2$ forment ensemble un radical de formule $-(CH_2)_n-$ (avec n = 4,5 ou 6), $-(CH_2)_2-O-(CH_2)_2-$, $-(CH_2)_2-$

$N-(CH_2)_2-$
|
$CH_3$

ainsi que leurs sels avec des acides minéraux ou organiques pharmacologiquement acceptables.

Les sels peuvent être notamment ceux formés avec les acides chlorhydrique, sulfurique, phosphorique, méthane-sulfonique, maleique, succinique, pamoique, acétique, fumariqe, lactique, aspartique et citrique.

Les composés de formule I peuvent être préparés selon la schéma suivant:

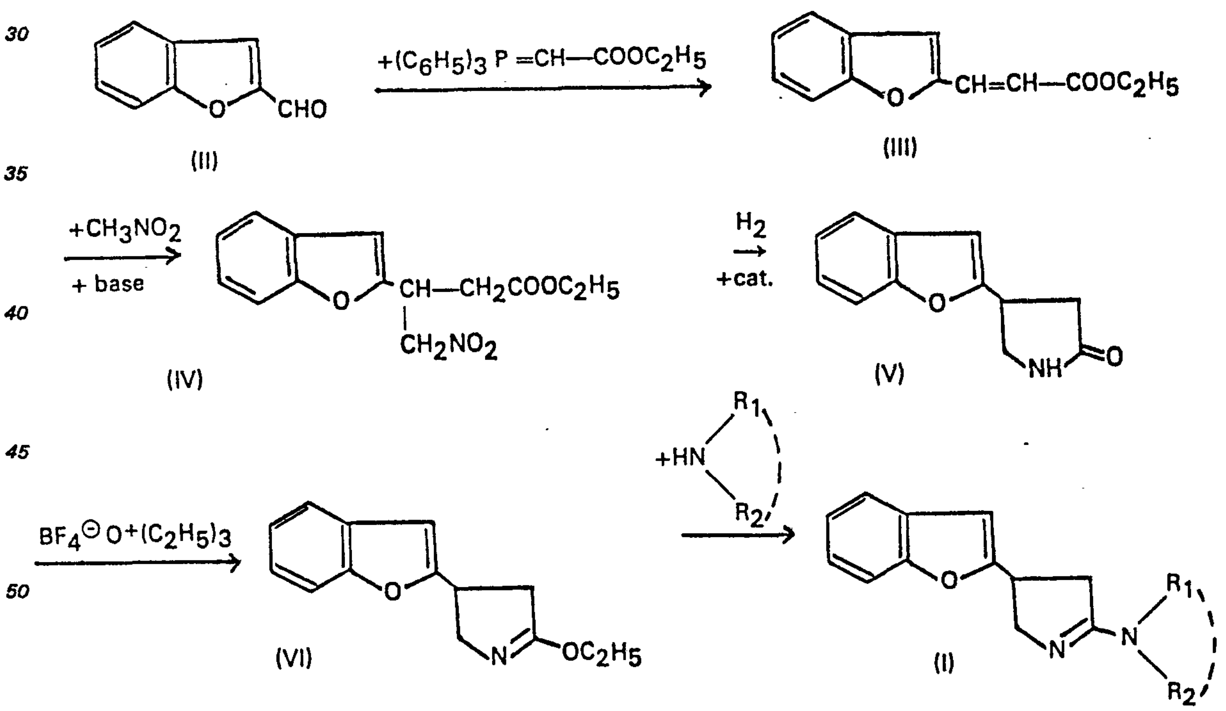


(II) (III) (IV) (V) (VI) (I)

La 1ère réaction consiste à faire réagir le formyl-2 benzofuranne (Bull. Soc. Chim. France 1962, p. 1875) avec le carbéthoxyméthylidène-triphényl-phosphorane, dans les conditions habituelles de la réaction classique de Wittig, au sein d'un solvant inerte tel que le benzène ou le toluène.

On obtient ainsi le (benzofuryl-2) -3 acrylate d'éthyle (III) qui a déjà été décrit par Foo Pan et Tsan Ching Wang (J. Chinese Chem. Soc.1961, série II, 8, 374—9).

On fait réagir l'acrylate (III) ainsi obtenu avec du nitro-méthane (généralement utilisé comme solvent de la réaction) en présence d'une base forte telle que le Triton B (hydroxyde de benzyltriméthylammonium) ou un alcoolate métallique tel que l'éthylate ou le méthylate de sodium, afin d'obtenir l'ester nitré (IV). On peut également effectuer la réaction dans un autre solvant tel que le diméthylformamide.

L'ester nitré (IV) est réduit par hydrogénation, sous pression ordinaire en présence d'un catalyseur tel que le Nickel de Raney dans un solvant tel que l'éthanol; l'amino-ester formé intermédiairement est cyclisé par chauffage en pyrrolidinone-2 (V).

La pyrrolidinone (V) est traitée par le tétrafluoborate de triéthyloxonium au sein d'un solvant inerte tel que le chlorure de méthylène et transformé en éthoxy-2 pyrroline (VI).

Sur cette dernière on fait réagir une amine $HNR_1R_2$ dans un solvant tel que l'éthanol, pour obtenir l'amino-2 pyrroline (I), transformée ensuite éventuellement en un sel par les moyens habituels. On peut aussi dans le cas des amines volatiles les utiliser sous forme de chlorhydrates, et parvenir ainsi directement aux chlorhydrates des amino-2 pyrrolines (I).

Exemple n° 1

(I) $R_1 = R_2 = H$

a) (Benzofuryl-2)-3 acrylate d'éthyle (III)

Un mélange de 35 g (0,24 mole) de formyl-2 benzofuranne (II) et de 83,5 g (0,24 mole) de carbéthoxyméthylidène-triphényl-phosphorane dans 300 ml de benzène est porté à reflux sous azote, pendant 7 heures. Après évaporation à sec, sous pression réduite, le résidu est trituré avec de l'éther, essoré et recristallisé dans l'éther isopropylique.

$F_{(inst.)}$ 78°C — Rend$^t$ ≃ 100%

Le produit ainsi obtenu peut contenir un peu d'oxyde de triphénylphosphine formé au cours de la réaction. Il peut en être débarrassé par distillation ($eb_{0.2}$ = 126°C ou $eb_{0,5}$ = 140°C Rend$^t$ 80%)

b) Nitro-4 (benzofuryl-2) -3 butyrate d'éthyle (IV)

51,8 g (0,24 mole) du dérivé (III) obtenu en a), 61 g (1 mole de nitrométhane et 10 ml de Triton B en solution à 40% dans le méthanol, sont portés à 70—90°C pendant 16 à 24 heures. Après refroidissement et acidification lente par l'acide chlorhydrique normal, l'ester nitré est extrait à l'éther, lavé à l'eau, séché sur sulfate de sodium anhydre. L'évaporation de l'éther laisse un résidu huileux de nitro-ester, purifié par distillation rapide.

$eb_{0,1}$ = 150°C ou $eb_{0,6}$ = 178°C

Les fractions de tête contenant de l'ester (III) qui n'a pas réagi, peut être recyclé dans une autre opération (Rend$^t$ moyen, sur 3 opérations successives: 69%).

c) (benzofuryl-2)-4 pyrrolidinone-2 (V)

L'ester IV (81,5 g- 0,297 mole) obtenu en b) est dissous dans 500 ml d'éthanol et hydrogéné sous pression ordinaire à 55°C, en présence de 10 g de Nickel de Raney. L'hydrogène nécessaire à la réaction (14,8 l) est absorbé en 7 heures. Après filtration du catalyseur et évaporation de l'éthanol, le résidu huileux est chauffé à 100°C sous vide de la trompe à eau, pendant 2 h. Par trituration avec de l'éther isopropylique, le produit cristallise —$F_{(inst)}$ 142°C — pds: 47,9 g (80%).

d) Ethoxy-2 -benzofuryl-2)-4 Δ-1 pyrroline (VI)

8 g (0,04 mole) de (benzofuryl-2) -4 pyrrolidinone-2 (V) et 12 g (0,06 mole) de tétrafluoroborate de triéthyloxonium sont dissous dans 80 ml de chlorure de méthylène et agités pendant une semaine à température ordinaire. Le mélange est ensuite hydrolysé par addition de 15 g de potasse dissous dans 30 ml d'eau. L'insoluble est filtré et la phase organique est lavée à l'eau, séchée sur sulfate de soude, et distillée — $eb_{0,3}$ = 128 — 130°C — poids: 5,9 g (64%).

e) Amino-2 (benzofuryl-2)-4 Δ-1 pyrroline, chlorhydrate

Les 5,9 g (0,025 mole) de dérivé (VI) obtenus dans la réaction précédente sont dissous dans 50 ml d'éthanol, avec 1,36 g (0,025 mole) de chlorure d'ammonium. La solution est portée à reflux pendant 20 h. et filtrée à chaud. Par refroidissement, le chlorhydrate du dérivé aminé cherché cristallise. Il est essoré et séché sous vide — $F_{(inst)}$ = 253°C — poids 4 g. (42%)

Exemple n° 2

(I) $R_1 = H, R_2 = CH_3$

Méthylamino-2 (benzofuryl-2) Δ-1 pyrroline, chlorhydrate

Il est obtenu comme le dérivé (I) de l'exemple 1 par action du chlorhydrate de méthylamine sur l'éthoxy-2 (benzofuryl-2) -4 Δ-1 pyrroline (VI), dans l'éthanol à ébullition. La solution est ensuite évaporée à sec et le résidu est repris par l'isopropanol chaud. Après addition d'acétate d'éthyle et agitation 24 h à température ordinaire, le précipité est essoré, et séché, $F_{(inst.)}$: 151 °C — Rend$^t$ 45%.

Exemple n° 3

(I) $R_1 = H, R_2 = iC_3H_7$

Isopropylamino-2 (benzofuryl-2) Δ-1 pyrroline, chlorhydrate

Il est obtenu comme le dérivé (I) de l'exemple 1 par action du chlorhydrate d'isopropylamine sur le dérivé (VI) dans l'éthanol à ébullition. Après évaporation du solvant, le résidu est repris par l'isopropanol chaud: le chlorhydrate cherché cristallise par addition d'éther isopropylique et refroidissement.

$F_{(inst.)}$: 186°C — Rend$^t$ = 66%

Exemple n°4

(I) $R_1 = R_2 = CH_3$

Diméthylamino-2 (benzofuryl-2)-4 Δ-1 pyrroline, chlorhydrate

Il est obtenu, comme le dérivé (I) de l'exemple 1, par action du chlorhydrate de diméthylamine sur l'éthoxy-2 (benzofuryl-2)-4 Δ1 pyrroline (VI), dans l'éthanol à chaud. Après évaporation à sec et reprise par l'isopropanol, le chlorhydrate cristallise par addition d'éther.

— $F_{(inst.)}$: 154°C — Rend$^t$: 84%

Exemple n° 5

(I) $R_1 = R_2 = C_2H_5$

Diéthylamino-2 (benzofuryl-2)-4 Δ-1 pyrroline, fumarate

Le chlorhydrate est obtenu comme dans les exemples précédents par action du chlorhydrate de diéthylamine sur le dérivé (VI), dans le butanol à ébullition (20 h). Après évaporation du solvant, le résidu sec est repris par le chloroforme, et la solution est agitée avec de la soude N, puis lavée à l'eau. Le résidu huileux laissé par l'évaporation du chloroforme est distillé sous pression réduite. $eb_{0,1}$ = 162°C — 168°C.

La base ainsi obtenue est dissoute dans du propanol, avec la quantité stoechiométrique d'acide fumarique à chaud. Par refroidissement, le fumarate cristallise.

$F_{(inst.)}$: 154°C — Rend$^t$: 35%

Exemple n° 6

(I) $R_1 = H, R_2 = C_6H_5$

Anilino-2 (benzofuryl-2)-4 Δ- pyrroline, chlorhydrate

La base est préparée par action d'un excès d'aniline sur le dérive (VI), dans l'éthanol à reflux (48 h). Après évaporation sous pression réduite, le résidu est repris par l'acétate d'éthyle, au sein duquel la base cristallise par agitation.

Celle-ci est transformée en chlorhydrate par addition d'une solution d'HCl anhydre dans l'éthanol à une solution de la base dans l'isopropanol, à chaud. Le chlorhydrate cristallise par refroidissement.

$F_{(inst.)}$: 203°C — Rend$^t$: 58%

Exemple n° 7

(I) $R_1 = H, R_2 = CH_2CH_2C_6H_5$

Phénéthylamino-2 (benzofuryl-2)-4 Δ -1 pyrroline, chlorhydrate

La base est obtenue comme celle du dérivé de l'exemple 6, à partir de phénéthylamine dans l'éthanol, puis transformée en chlorhydrate — $F_{(inst.)}$: 212°C — Rend$^t$: 62%

Exemple n° 8

(I) $R_1—R_2 = —(CH_2)_4—$

Pyrrolidino-2 (benzofuryl-2)-4 Δ-1 pyrroline, chlorhydrate

Il est obtenu, comme le dérivé (I) de l'exemple 1, par action du chlorhydrate de pyrrolidine sur le dérivé (VI), dans l'éthanol à chaud. Après évaporation du solvant et redissolution dans l'isopropanol chaud, le chlorhydrate cristallise par addition d'éther isopropylique et refroidissement.

$F_{(inst.)}$ 260°C Rend$^t$: 84%

Exemple n° 9

(I) $R_1—R_2 = —(CH_2)_2—O—(CH_2)_2—$

Morpholino-2 (benzofuryl-2) -4Δ-1 pyrroline, chlorhydrate

Il est obtenu, comme le dérivé (I) de l'exemple 1 par action du chlorhydrate de morpholine sur le dérivé (VI) dans l'éthanol à chaud, puis recristallisation dans l'isopropanol.

$F_{(inst.)}$ = 195°C — Rend$^t$: 81%

Exemple n° 10

(I) $—R_1—R_2 = —(CH_2)_2—N(CH_3)—(CH_2)_2—$

(Méthyl-4-pipérazino)-2 (benzofuryl-2)-4Δ-1-pyrroline, méthanesulfonate

Il est obtenu, comme le dérivé (I) de l'exemple n°6, par action de la N-méthylpipérazine sur le dérivé (VI) dans l'éthanol, à chaud. Après évaporation sous pression réduite le résidu est trituré avec de l'éther de pétrole, essoré et séché.

$F_{(inst.)}$: 124°, 5 — Rend$^t$ 58%.

La base est transformée en méthane-sulfonate par addition d'acide méthanesulfonique en léger défaut, dans l'acétate d'éthyle, et agitation pendant 3 h. Le sel insoluble est essoré et séché.

F: 146°C — Rend$^t$: 79,5%.

Exemple n° 11

(I) $R_1 = H, R_2 = CH_2CH_2\ N(CH_3)_2$

(Diméthylamino-2 éthylamino)-2 (benzofuryl-2)-4 Δ-1 pyrroline, dichlorhydrate.

Il est obtenu, comme le dérivé de l'exemple 6, par action de la diméthylamino-2 éthylamine sur le dérivé VI, à chaud dans l'éthanol, puis transformation de la base en dichlorhydrate dans l'isopropanol. Le dichlorhydrate cristallilse par refroidissement.

$F_{(inst.)}$: 240°C — Rend$^t$: 52%

Exemple n° 12

(I) $R_1 = H, R_2 = OCH_2C_6H_5$

Benzyloxyamino-2 (benzofuryl-2)-4 Δ-1 pyrroline, chlorhydrate

Il est obtenu comme le dérivé (I) de l'exemple 1, par action du chlorhydrate de benzyloxyamine sur le dérivé (VI) dans l'éthanol à chaud. Après évaporation du solvant et redissolution dans l'isopropanol chaud, le chlorhydrate cristallise par addition d'éther isopropylique et refroidissement.

$F_{(inst.)}$ = 183°C — Rend$^t$: 39%.

Exemple n° 13

(I) $R_1 = H, R_2 = OCH_3$

Méthoxyamino-2 (benzofuryl-2)-4 Δ-1 pyrroline, chlorhydrate

Il est obtenu, comme le dérivé (I) de l'exemple n°1, par action du chlorhydrate de O-méthylhydroxylamine sur le dérivé (VI) dans l'éthanol chaud.

$F_{(inst.)}$ = 184°C (avec dec) — Rend$^t$ = 67%.

Exemple n° 14

(I) $R_1 = H, R_2 = CH_2CH = CH_2$

Allylamino-2 (benzofuryl-2)-4 Δ-1 pyrroline, chlorhydrate

Il est obtenu, comme le dérivé (I) de l'exemple n° 6, par action de l'allylamine sur le composé (VI), dans l'éthanol à chaud. Après évaporation à sec, le résidu est transformé en chlorhydrate par addition d'HCl en solution dans l'éther anhydre.

F: 160°C — Rend$^t$ = 98%

Exemple n° 15

(I) $R_1 = H, R_2 = CH_2C \equiv CH$

Propargylamino-2 (benzofuryl-2)-4 Δ-1 pyrroline, chlorhydrate

Il est obtenu, comme le dérivé (I) de l'exemple n°1, par action du chlorhydrate de propargylamine sur le composé (VI) dans l'éthanol à chaud. Après évaporation du solvant, le résidu est recristallisé dans l'isopropanol.

F: 196°C — Rend$^t$ = 75%

Exemple n° 16

(I) $R_1 = H, R_2 = CH_2CH_2$ 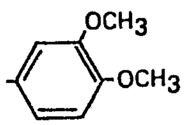

Dimethoxy-3,4 phenethylamino)-2 benzofuryl-2)-4 Δ-1 pyrroline, chlorhydrate

Il est obtenu, comme le dérivé (I) de l'exemple n°1, par action du chlorhydrate de (diméthoxy-3,4 phényl)-2 éthylamine sur le composé (VI) dans l'éthanol à chaud. Après évaporation du solvant, le résidu est recristallisé dans l'isopropanol.

F: 180°C — Rend$^t$ = 76%

Exemple n° 17

(I) $R_1 = H, R_2 = CH_2CH_2OH$

(Hydroxy-2 éthylamino)-2 (benzofuryl-2)-4 Δ-1 pyrroline, chlorhydrate.

Il est obtenu, comme le dérivé (I) de l'exemple n°6, par action de l'éthanolamine sur le composé (VI) dans l'éthanol à chaud. La base isolée après évaporation de l'éthanol (F: 130°C — Rend$^t$ 69%) est transformée en chlorhydrate par une solution d'HCl dans l'ether anhydre.

F: 171°C — Rend$^t$ = 82%

Exemple n° 18

(I) $R_1 = H, R_2 = CH_2COOC_2H_5$

(Ethoxycarbonyl-2-méthylamino)-2 (benzofuryl-2)-4 Δ-1 pyrroline, chlorhydrate

Il est obtenu, comme le dérivé (I) de l'exemple n°1, par action du chlorhydrate d'amino-2 acétate d'éthyle sur le composé (VI), dans l'éthanol à chaud.

F: 168°C — Rend$^t$ = 69%

Les composés de formule I présentent des propriétés pharmacologiques intéressantes particulièrement dans le domaine cardiovasculaire: augmentation du débit sanguin artériel et action antidysrythmique. Leur toxicité n'apparaît qu'à des doses très supérieures aux doses pharmacologiquement actives, ce qui permet leur utilisation en thérapeutique comme médicaments pour le traitement des dysrythmies et l'amélioration de la circulation sanguine.

On donnera ci-après des résultats des études toxicologiques et pharmacologiques qui mettent en évidence ces propriétés.

a) *toxicité aiguë chez la souris*

Chaque composé a été administré par voie orale, intra-péritonéale, ou intraveineuse, en une seule fois. Le comportement des animaux et la mortalité ont été observés pendant plusieurs heures après le traitement puis quotidiennement pendant au moins une semaine.

Les résultats sont donnés dans le tableau I ci-après.

TABLEAU I

| Exemple N° | $DL_{50}$ po | $DL_{50}$ ip | $DL_{50}$ iv | Observations |
|---|---|---|---|---|
| 1 | 115 mg/kg | 58 mg/kg | 46 mg/kg | Vasodilatation |
| 2 | >200 mg/kg | 58 mg/kg | | agitation-convulsions |
| 3 | 200 mg/kg | 58 mg/kg | | convulsions-dyspnée |
| 4 | 240 mg/kg | | 38,5 mg/kg | convulsions |
| 5 | 180 mg/kg | 58 mg/kg | | stimulation centrale |
| 6 | 110 mg/kg | 58 mg/kg | | hyperesthesies |
| 7 | >200 mg/kg | 98 mg/kg | | convulsions |
| 8 | 200 mg/kg | 76 mg/kg | | agitation-convulsions |
| 9 | >200 mg/kg | 142 mg/kg | | convulsions-cyanose |
| 10 | >200 mg/kg | 142 mg/kg | | agitation-convulsions |

b) *actions sur le système nerveux central*

Les effets centraux des différents composés ont été étudiés chez la souris au moyen d'un ensemble de tests
— test de la traction;
— intéraction avec l'effet hypnotique des barbituriques;
— test à l'oxotrémorine;
— test à la réserpine (ptosis).

Ces tests n'ont pas mis en évidence d'effet notable sur le système nerveux central.

c) *effets cardiovasculaires*

Les effets cardiovasculaires de chaque composé ont été étudiés chez le chien anesthésié.

Un effet dilatateur artériel puissant a été mis en évidence pour un certain nombre de composés.

d) *effets antidysrythmiques*

L'activité antidysrythmique des composés de formule I a été évaluée d'aprés les tests pharmacologiques suivants:

— fréquence cardiaque maximale d'entraînement (FMS).

La FMS mesurée in vivo chez le lapin par entraînement cardiaque au moyen d'une sonde de stimulation placée dans la cavité auriculaire gauche explore globalement la durée de la période réfractaire des voies de conduction et des fibres myocardiques.

— dysrythmies par stimulation centrale chez le lapin: la stimulation électrique de l'hypothalamus postérieur entraîne une activité intense du système sympathique et des dysrythmies;

— dysrythmies à l'aconitine chez le rat: cet agent perturbe profondément les perméabilités ioniques membranaires;

— dysrythmies à l'ouabaïne chez le cobaye: aux doses toxiques l'ouabaïne perturbe les échanges ioniques transmembranaires; d'autre part elle provoque une activation sympathique concomitante.

— dysrythmies par anoxie chez le rat, après prétraitement par une dose subliminaire d'aconitine (association des effets d'une stimulation sympathique à ceux des perturbations des conducteurs ioniques);

— méthode de Harris chez le chien: ligature coronaire et dysrythmies par ischémie localisée.

Les résultats obtenus figurent dans le tableau II. Ce tableau met en évidence une activité antidysrythmique puissante des composés de formule I.

TABLEAU II

Activité antidysrythmique

| Exemple N° | FMS Lapin | Stimulation centrale Lapin | Aconitine Rat | Anoxie Rat | Ouabaïne Cobaye | Ligature Harris Chien |
|---|---|---|---|---|---|---|
| 2 | + (7,5 mg/kg) | + (3 mg/kg) | + (10 mg/kg) | | | |
| 3 | | | 0 | + (10 mg/kg) | | |
| 4 | + (2,5 mg/kg) | ++ (5 mg/kg) | ++ (10–20 mg/kg) | ++ (10–20 mg/kg) | + (10–20 mg/kg) | + (10 mg/kg) |
| 5 | + | | ++ (10–20 mg/kg) | | | |
| 6 | + (2,5 mg/kg) | + (2,5 mg/kg) | | 0 | | |
| 7 | + (2 mg/kg) | 0 | + (5 mg/kg) | | | |
| 8 | | | + (10 mg/kg) | + (10 mg/kg) | | |
| 9 | | | ± (10 mg/kg) | 0 | | |
| 10 | | | 0 | + (10 mg/kg) | | |

dose i.v. 0 : sans effet

+ : action modérée

++ : action nette

Les composés de formule I présentent un intérêt thérapeutique important dans le traitement de diverses maladies circulatoires ou cardiaques (insuffisances circulatoires, obturations vasculaires, angor, dysrythmies, etc..)

Les composés sont administrables à l'homme par voie orale ou rectale à l'état de bases ou de sels (en comprimés, gélules, gouttes ou suppositoires) ou par voie parentérale sous forme de solution aqueuse d'un sel hydrosoluble ou au sein d'un autre excipient assurant une résorption différée. Les différentes présentations peuvent contenir de 10 à 1 000 mg de principe actif par unité de prise pour l'administration par les voies orale et rectale, et de 5 à 500 mg de principe actif pour les autres voies d'administration.

La posologie journalière peut varier, de 10 mg à 3 g, selon les voies d'administration et les indications thérapeutiques envisagées.

## Revendications

1. Composés de formule:

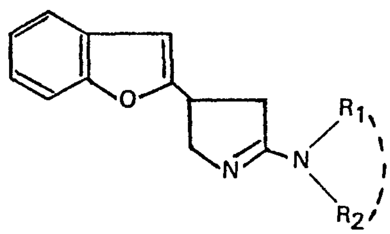

I

dans laquelle $R_1$ est un atome d'hydrogène et $R_2$ un atome d'hydrogène, un radical alcoyle en $C_1$—$C_6$, alcényle en $C_2$—$C_6$, alcynyle en $C_2$—$C_6$, phényle, phénylalcoyle ($C_1$—$C_6$), mono, di ou tri-méthoxyphénylalcoyle ($C_1$—$C_6$), alcoxy en $C_1$—$C_6$, benzyloxy, dialcoyl ($C_1$—$C_6$)amino-alcoyle ($C_1$—$C_6$), hydroxalcoyle ($C_1$—$C_6$), alcoyl ($C_1$—$C_6$) oxycarbonyl alcoyle ($C_1$—$C_6$) ou bien $R_1$ et $R_2$ sont des radicaux alcoyle en $C_1$—$C_6$ ou bien $R_1$ et $R_2$ forment ensemble un radical de formule $-(CH_2)_n-$ (avec n = 4,5 ou 6), $-(CH_2)_2-O-(CH_2)_2-$, $-(CH_2)_2-\underset{\underset{CH_3}{|}}{N}-(CH_2)_2-$

ainsi que leurs sels avec des acides minéraux ou organiques pharmacologiquement acceptables.

2. Composés selon la revendication 1, caractérisés en ce que $R_1$ et $R_2$ sont des groupes alcoyles en $C_1$—$C_6$.

3. Composés selon la revendication 2, caractérisé en ce que $R_1$ et $R_2$ sont des groupes méthyle.

4. Procédé de préparation des composés de formule I telle que définie à la revendication 1, caractérisé en ce que a) on fait réagir un acrylate de formule:

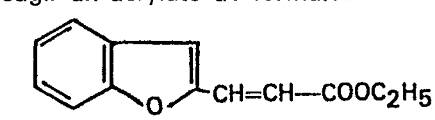

III

avec du nitrométhane en présence d'une base forte, obtenant ainsi un ester nitré de formule

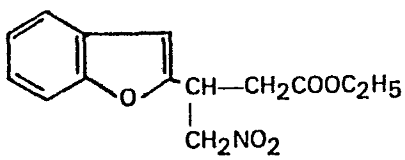

IV

b) on réduit l'ester nitré ainsi obtenu par hydrogénation en présence d'un catalyseur, obtenant ainsi un amino-ester que l'on cyclise par chauffage en une pyrrolidinone-2 de formule:

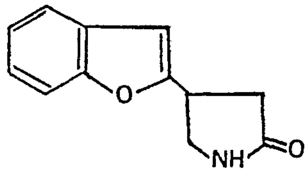

V

c) on fait réagir la pyrrolidinone-2 ainsi formée avec du tétrafluorborate de triéthyloxonium au sein d'un solvant inerte, obtenant ainsi une éthoxy-2 pyrroline de formule:

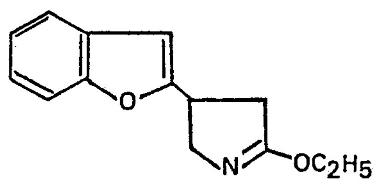

VI

d) on fait réagir l'éthoxy-2 pyrroline ainsi formée sur une amine de formule $HNR_1R_2$ dans laquelle $R_1$ et $R_2$ ont les significations données à la revendication 1, obtenant ainsi un composé de formule I et, e) on transforme éventuellement le composé obtenu en un sel.

5. Procédé selon la revendication 4, caractérisé en ce que pour préparer le composé de formule III, on fait réagir le formyl -2 benzofuranne avec le carbéthoxyméthylidène-triphényl-phosphorane dans les conditions de la réaction de Wittig.

6. Composition thérapeutique, caractérisée en ce qu'elle contient à titre de principe actif un composé selon l'une quelconque des revendications 1 à 3.

**Claims**

1. Compounds having the formula:

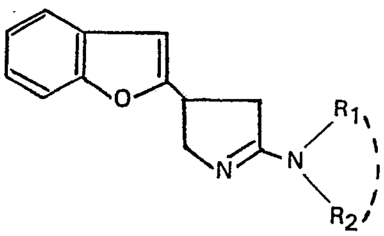

(I)

in which $R_1$ is a hydrogen atom and $R_2$ is a hydrogen atom, a $C_{1-6}$ alkyl, $C_{2-6}$ alkenyl, $C_{2-6}$ alkynyl, phenyl, phenyl $C_{1-6}$ alkyl, mono, di or tri-methoxyphenyl $C_{1-6}$ alkyl, $C_{1-6}$ alkoxy, benzyloxy, $C_{1-6}$ dialkyl amino $C_{1-6}$ alkyl, $C_{1-6}$ hydroxyalkyl, $C_{1-6}$ alkyl oxycarbonyl $C_{1-6}$ alkyl or $R_1$ and $R_2$ are $C_{1-6}$ alkyl radicals or $R_1$ and $R_2$ form together a radical of the formula
$-(CH_2)_n-$ (with n = 4, 5 or 6), $-(CH_2)_2-O-(CH_2)_2-$, $-(CH_2)_2-\underset{CH_3}{\underset{|}{N}}-(CH_2)_2-$

and their salts with pharmacologically acceptable inorganic or organic acids.

2. Compounds as claimed in claim 1, wherein $R_1$ and $R_2$ are $C_{1-6}$ alkyl groups.

3. Compounds as claimed in claim 2, wherein $R_1$ and $R_2$ are methyl groups.

4. Process for the preparation of compounds of the formula (I) as defined in claim 1, characterized in that it comprises
a) reacting an acrylate having the formula:

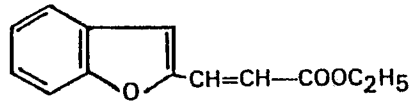

(III)

with nitromethane, in the presence of a strong base, to give a nitro ester having the formula:

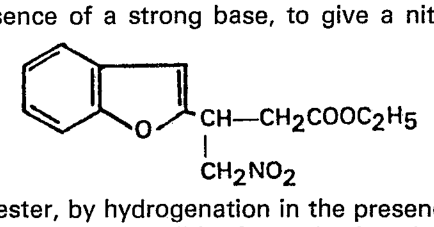

(IV)

b) reducing the resulting nitro ester, by hydrogenation in the presence of a catalyst, to give an amino-ester which is cyclized by heating to a pyrrolidin-2-one having the formula:

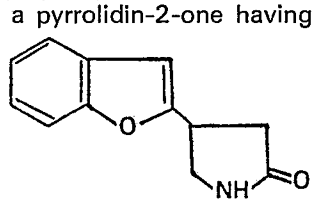

(V)

c) reacting the resulting pyrrolidin-2-one with triethyloxonium tetrafluoborate within an inert solvent, to give an 2-ethoxy-pyrroline having the formula:

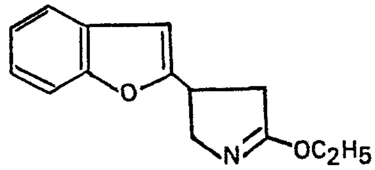

(VI)

d) reacting the resulting 2-ethoxy-pyrroline with an amine having the formula $HNR_1R_2$ in which $R_1$ and $R_2$ are as defined in claim 1, to give a compound of the formula (I), and

e) optionally converting the resulting compound to a salt.

5. Process as claimed in claim 4, wherein, to prepare the compound of the formula (III), 2-formylbenzofuran is reacted with carbethoxymethylidene-triphenyl-phosphoran under Wittig reaction conditions.

6. A therapeutic composition characterized in that it contains as active ingredient a compound as claimed in any one of claims 1 to 3.

**Patentansprüche**

1. Verbindungen der Formel

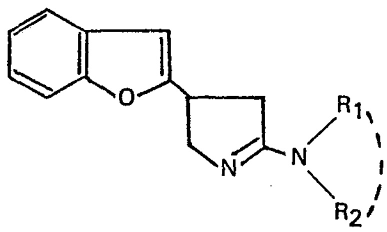

I

in der $R_1$ ein Wasserstoffatom und $R_2$ ein Wasserstoffatom, ein $C_1$—$C_6$-Alkylrest, ein $C_2$—$C_6$-Alkenylrest, ein $C_2$—$C_6$-Alkinylrest, ein Phenylrest, ein Phenylalkylrest ($C_1$—$C_6$), ein Mono- Di- oder Trimethoxyphenylalkylrest ($C_1$—$C_6$), ein $C_1$—$C_6$-Alkoxyrest, ein Benzyloxyrest, ein Dialkyl-($C_1$—$C_6$)-aminoalkylrest ($C_1$—$C_6$), ein Hydroxyalkylrest ($C_1$—$C_6$), ein Alkyl($C_1$—$C_6$)-oxycarbonylalkylrest ($C_1$—$C_6$) ist oder $R_1$ und $R_2$ $C_1$—$C_6$-Alkylreste sind oder $R_1$ und $R_2$ gemeinsam einen Rest der Formel $—(CH_2)_n—$ (worin n = 4,5 oder 6), $—(CH_2)_2—O—(CH_2)_2—$, $—(CH_2)_2—N—(CH_2)_2—$

$|$

$CH_3$

bilden, und ihre Salze mit pharmakologisch unbedenklichen Mineralsäuren oder organischen Säuren.

2. Verbindungen nach Anspruch 1, dadurch gekennzeichnet, daß $R_1$ und $R_2$ $C_1$—$C_6$-Alkylreste sind.

3. Verbindungen nach Anspruch 2, dadurch gekennzeichnet, daß $R_1$ und $R_2$ Methylreste sind.

4. Verfahren zur Herstellung von Verbindungen der Formel I, wie sie in Anspruch 1 definiert ist, dadurch gekennzeichnet, daß man

a) ein Acrylat der Formel

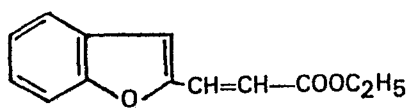

III

mit Nitromethan in Gegenwart einer starken Base umsetzt und hierbei einen nitrierten Ester der Formel

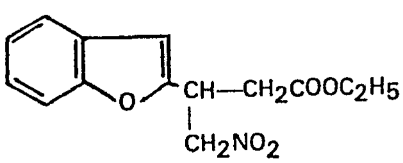

IV

bildet,

b) den hierbei erhaltenen nitrierten Ester durch Hydrierung in Gegenwart eines Katalysators reduziert und so einen Aminoester erhält, den man durch Erhitzen zu einem Pyrrolidinon-2 der Formel

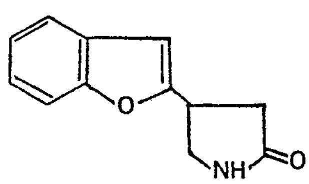

V

cyclisiert,

c) das so gebildete Pyrrolidinon-2 mit Triäthyloxoniumtetrafluorborat in einem inerten Lösungsmittel umsetzt und so ein 2-Äthoxypyrrolin der Formel

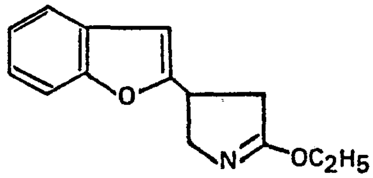

VI

erhält,

d) das so gebildete 2-Äthoxypyrrolin mit einem Amin der Formel $HNR_1R_2$, in der $R_1$ und $R_2$ die in Anspruch 1 genannten Bedeutungen haben, umsetzt und

e) die erhaltene Verbindung gegebenenfalls in eine Salz umwandelt.

5. Verfahren nach Anspruch 4, dadurch gekennzeichnet, daß man zur Herstellung der Verbindung der Formel III 2-Formylbenzofuran mit Carbäthoxymethyliden-triphenylphosphoran unter den Bedingungen der Wittig-Reaktion umsetzt.

6. Arzneimittelzubereitung, dadurch gekennzeichnet, daß sie als Wirkstoff eine Verbindung nach einem beliebigen der Ansprüche 1 bis 3 enthält.